# EUROPÄISCHE PATENTSCHRIFT

(11) **EP 1 187 556 B1**
(45) Veröffentlichungstag und Bekanntmachung des Hinweises auf die Patenterteilung: **29.03.2006**
(21) Anmeldenummer: 00929191.5
(22) Anmeldetag: 02.06.2000
(51) Int. Cl.: A61B 17/02

(54) **BÄNDERSPANNVORRICHTUNG FÜR NICHT-KUGELIGE GELENKE**
LIGAMENT-TENSIONING DEVICE FOR NON-SPHEROID JOINTS
DISPOSITIF DE TENSION DE LIGAMENTS POUR DES ARTICULATIONS NON ORBICULAIRES

(30) Priorität: 19.06.1999 DE 29910761 U
(43) Veröffentlichungstag der Anmeldung: 20.03.2002
(73) Patentinhaber: Mathys AG Bettlach, 2544 Bettlach (CH)
(72) Erfinder: MOSER, Walter, CH-3126 Kaufdorf (CH); WEHRLI, Ulrich, CH-3084 Wabern (CH)
(74) Vertreter: Lusuardi, Werther
(86) Internationale Anmeldenummer: PCT/CH2000/000308
(87) Internationale Veröffentlichungsnummer: WO 2000/078225

(56) Entgegenhaltungen:
- WO-A-96/17552
- US-A- 3 750 652
- US-A- 4 899 761
- US-A- 4 997 432
- US-A- 5 213 112

## Beschreibung

Die Erfindung bezieht sich auf eine Vorrichtung zum Spannen von Bändern an nicht-kugeligen Gelenken am menschlichen oder tierischen Körper gemäss dem Oberbegriff des Patentanspruchs 1.

Die erfindungsgemässe Vorrichtung betrifft damit ein chirurgisches Instrument, welches es erlaubt, Gelenkendoprothesen mit einer vorbestimmten Spannung der das Gelenk steuernden Bänder und Kapselstrukturen zu implantieren.

Voraussetzung für eine gute Funktion des prothetisch versorgten Gelenks ist das konfliktfreie Zusammenspiel der Kinematik des Kunstgelenks und der das Gelenk umgebenden Weichteile. Bei nicht kugelförmigen Gelenken, wie z.B. dem Kniegelenk oder dem Ellbogengelenk, wird die Gelenkbewegung durch die umgebenden Kapsel-Bandstrukturen gesteuert. Beim natürlichen Gelenk sind die Geometrie der Artikulationsflächen und der Kapsel-Bandapparat so aufeinander abgestimmt, daß die physiologische Bewegung möglich ist. Beim Ersatz des erkrankten natürlichen Gelenkes durch ein Kunstgelenk ist die Abstimmung der Kinematik des Kunstgelenkes mit dem Kapsel-Bandapparat bestimmend für die Beweglichkeit und Stabilität des versorgten Gelenks. Die natürlichen Strukturen der Kapsel und Bänder beinhalten Rezeptoren zur Erzeugung neurologischer Signale für die Steuerung der Muskeln der Extremität. Diese Rezeptoren setzen mechanische Reize, welche durch Dehnungen des umgebenden Gewebes erzeugt werden, in neurologische Signale um. Je nach Einbausituation des Kunstgelenkes ist dieses Informationssystem mehr oder weniger geschädigt, und der Patient hat somit Einbussen an der Propriozeptivität der Extremität.

Patienten, bei welchen bei der Implantation eine gute Abstimmung von Weichteilspannung über den Bewegungsumfang des Kunstgelenkes erzielt wurde, weisen eine bessere Propriozeptivität auf, als solche, bei denen eine weniger gute Abstimmung erzielt wurde (The Journal of Bone and Joint Surgery, Vol 73-B, Jan. 1991 und Vol. 78-B, July 1996).

Diese Erkenntnis wurde anhand des Kniegelenkersatzes erarbeitet und diese neuzeitlichen Operationstechniken sind beispielsweise in der EP 0 322 363 WEHRLI und der WO 96/17552 TODD beschrieben. Diese Operationstechniken werden durch ein Instrumentarium unterstützt, welches es erlaubt, die Statik des zu operierenden Beines mit hoher Genauigkeit wiederherzustellen und das Kunstgelenk sicher und dauerhaft am Skelett zu verankern. Zusätzlich zur Wiederherstellung der schmerzfreien Beweglichkeit ist die Ausrichtung der Extremität bezüglich der Achse (varus - valgus) und der Rotationsposition (Innen-/Außenrotation) ein wichtiges Ziel der Operation. Der heutige Stand der Instrumentarien erlaubt in hohem Masse die Reproduktion der korrekten Achsverhältnisse. In der Regel wird die Weichteilsituation chirurgisch auf das Kunstgelenk adaptiert, jedoch wird keine Quantifizierung der Spannung der das Gelenk passiv steuernden Weichteilstrukturen vorgenommen. Führende Größe in den beschriebenen Operationstechniken ist die statikgerechte Positionierung des Kunstgelenkes. Die Weichteilsituation wird nach wiederhergestellter Statik durch sogenannte Kapsel- und Bandreleases korrigiert, wobei diese Korrektur in verschiedenen Beugestellungen des Gelenkes vorgenommen wird.

Von verschiedenen Autoren wurden Instrumente vorgeschlagen, die eine kontrollierte Bearbeitung der Kapsel-Bandstrukturen während der Implantation eines Kunstgelenkes zum Ziel haben. Alle diese Instrumente haben eine Spreizerfunktion, die unterschiedlich technisch gelöst ist.

Ein Spreizinstrument für die Spannung der Bandstrukturen im Knie bei der Implantation einer Knieprothese ist in der US 5,649,929 CALLAWAY offenbart. Das Instrument basiert auf einem scherenartigen Konzept und erlaubt das Aufspreizen des Kniegelenks in Beugung. Dabei stützt sich ein Schenkel des Instrumentes auf der proximalen Tibia ab, und der zweite Schenkel wird in das gebeugte Knie zentral eingeführt. Die Spreizbewegung kann durch eine Spindel, die zwischen den Handgriffen angeordnet ist, arretiert werden. Das Instrument ist ausschließlich für die Verwendung im gebeugten Knie vorgesehen.

In der EP 0 322 363 WEHRLI wird ein Bänderspanninstrument offenbart, welches ebenfalls mittels zweier scherenförmig angeordneter Hebel eine Spreizwirkung erzielt, die mit einer zwischen den Handhebeln angeordneten Spindel mit Mutter arretiert werden kann. Das Instrument stützt sich tibial auf einer unterhalb der Gelenkfläche eingebrachten Knochenschraube ab und wird in der Mitte in das gebeugte Knie eingeführt und dann aufgespreizt. In der selben Patentschrift ist für die Spannung der Bänder am gestreckten Knie ein Instrument beschrieben, welches mittels zweier Gewindespindeln zwei in einer Längsführung geführte Spreizer antreibt. Die beiden getrennt bedienbaren Spreizer ermöglichen die getrennte Aufspreizung der beiden Gelenkkompartimente des Kniegelenkes. Dieser Doppelspreizer erzeugt gegenüber dem erstbeschriebenen eine parallele Spreizbewegung. Dadurch wird eine Spreizbewegung erzeugt, die bei unterschiedlichen Spreizwegen keine Winkeländerung der Spreizer ergibt, wie dies beim scherenförmigen Typ der Fall ist.
In der WO 96/17552 TODD ist ein Bänderspanner für die Implantation von Knieprothesen offenbart, der zwei getrennte Spreizer aufweist, die ebenfalls in einer Längsführung geführt sind. Der Antrieb erfolgt durch zwei Handhebel, wobei die Spreizposition durch einen Klinkenmechanismus arretiert werden kann. Handhebelbetriebene Spreizer bieten gegenüber den durch Gewindespindeln betriebenen Spreizer den Vorteil, daß der Chirurg eine direkte Kontrolle der Spreizkraft über die Handkraft hat. Je nach Hebelverhältnissen kann die Übersetzung der Handkraft in die Spreizkraft, ähnlich wie bei chirurgischen Zangen oder Scheren, frei gewählt werden. Kenntnisse und Training eines Chirurgen garantieren die sichere Handhabung derartiger Instrumente. Die manuell gefühlsmäßige Kontrolle der Spreizkraft über eine Gewindespindel ist deutlich weniger exakt und kann zur Überbeanspruchung der Weichteile führen. Nachteilig bei diesen erwähnten Spreizinstrumenten ist, daß zusätzlich zur gefühlsmäßigen Kontrolle keine Quantifizierung der Kraft, mit welcher die Weichteile gespannt sind, durch das Instrument möglich ist. Im weiteren ist aus der US 4,997,432 ein Spreizinstrument mit einem Viergelenk-Hebelgetriebe bekannt, welches allerdings keine Quantifizierung der angewandten Kraft erlaubt. Schliesslich ist aus der US 4,899,761 eine Vorrichtung bekannt, bei welcher mittels elektronischer Dehnmesstreifen die angewandte Kraft messbar ist, allerdings nur als Ganzes.
Hier will die Erfindung Abhilfe schaffen. Der Erfindung liegt die Aufgabe zugrunde, die Kapsel-Bandstrukturen eines prothetisch zu versorgenden Gelenkes mit einer parallelen Spreizbewegung anzuspannen, dabei eine manuelle Kontrolle und zusätzlich gleichzeitig eine quantitative Kontrolle der Spreizkraft zu ermöglichen. Das Instrument soll im gebeugten und gleichermaßen im gestreckten Gelenk verwendbar sein. Dazu dienen zwei getrennte Spreizer, welche mittels zweier Handhebelpaare bedient werden und zweier Viergelenkmechanismen parallel gespreizt werden. Zur Quantifizierung der Kraft pro Spreizer dient ein Blattfederelement, das über einen Zeiger und eine Skala die Spreizkraft in Newton anzeigt.

Die Erfindung löst die gestellte Aufgabe mit einer Vorrichtung zum Spannen von Bändern an nicht-kugeligen Gelenken, welche die Merkmale des Anspruchs 1 aufweist.

Weitere vorteilhafte Ausgestaltungen der Erfindung sind in den abhängigen Ansprüchen gekennzeichnet.

Die erfindungsgemässe Vorrichtung umfasst einen prismatischen, zylindrischen oder plattenförmigen Grundkörper und symmetrisch zu einer anterior-posterior verlaufenden Ebene angeordnet, zwei mit dem Grundkörper verbundene Pratzen, welche parallel auf die plan osteotomierte gelenkseitige Oberfläche eines ersten an ein nicht-kugeliges Gelenk angrenzenden Knochens zur Anlage bringbar sind, einen linken und einen rechten Handgriff, einen linken und einen rechten Bedienungshebel, welche gleichzeitig mit dem Halten der Vorrichtung mit je einer Hand am entsprechenden Handgriff einzeln mit der jeweils selben Hand betätigbar sind, und einen linken und einen rechten Spannhebel, welche mit ihren Auflageflächen auf die gelenkseitige Oberfläche eines zweiten an dass Gelenk angrenzenden Knochens zur Anlage bringbar sind. Die Bewegung der Spannhebel relativ zu den Pratzen erfolgt durch eine linke und eine rechte Parallelverschiebevorrichtung, welche je durch den entsprechenden Bedienungshebel antreibbar sind und so mit je einem Spannhebel verbunden sind, dass bei einer Bewegung der Bedienungshebel die Auflageflächen der Spannhebel unabhängig voneinander parallel zu den Auflageflächen an den Pratzen bewegbar sind. Die Parallelverschiebevorrichtungen sind als Viergelenk-Hebelgetriebe ausgestaltet. Die Gröf3e der Spreizkraft zwischen den Auflageflächen an den Pratzen und den Auflageflächen an den Spannhebeln ist an skalierten Kraftanzeigen ablesbar, wobei beim Spannen der Bänder durch Betätigung der Vorrichtung ein separates Ablesen der auf den linken beziehungsweise rechten Bedienungshebel ausgeübten Kraft möglich ist. Die Position der Spannhebel gegenüber den Pratzen ist mittels Feststellelementen lösbar arretierbar.

In einer bevorzugten Ausführungsform der erfindungsgemässen Vorrichtung enthält die Kraftanzeige einen beweglichen Anzeigehebel, dessen Kanten sich gegenüber der Skala verschieben. Bewegt wird dieser Anzeigehebel durch die longitudinale Biegung des einen, durch eine manuell aufgebrachte Spannkraft biegbaren Bedienungshebelteils gegenüber dem anderen, gabelartig angeordneten und nicht durch diese Spannkraft beaufschlagten Bedienungshebelteil. Werden mittels der Spannkraft die beiden Bedienungshebelteile relativ zueinander bewegt, verschiebt sich der Anzeigehebel gegenüber der Skala.

Die als Viergelenk-Hebelgetriebe ausgeführten Parallelverschiebevorrichtungen umfassen vorzugsweise jeweils vier Hebel, wobei ein oberer Hebel und ein unterer Hebel parallel angeordnet sind, der obere Hebel mit dem entsprechenden Spannhebel verbunden ist, der untere Hebel mit dem Grundkörper verbunden ist und die Verbindungshebel das Scherengestänge bilden, so dass der obere Hebel und der untere Hebel parallel zueinander oder voneinander weg bewegbar sind.

Die Hebellängen sind so gewählt, dass die Parallelverschiebevorrichtungen bezüglich der Kraftübertragung zwischen der mit der jeweiligen Hand zwischen den entsprechenden Bedienungshebel und Handgriffen ausgeübten Spannkraft und der von dem entsprechenden Spannhebel und der entsprechenden Pratze ausgeübten Distraktionskraft auf die an das Gelenk angrenzenden Knochen innerhalb einer Spannweite zwischen den Auflageflächen an den Pratzen und den Auflageflächen an den Spannhebeln zwischen 5 mm und 35 mm, vorzugsweise zwischen 7 mm und 25 mm ein Übersetzungsverhältnis von 1: 1 gewährleisten.

Die durch die Erfindung erreichten Vorteile sind im wesentlichen darin zu sehen, dass dank des erfindungsgemässen Bänderspanners während des Spannens der Bänder die auf diese ausgeübten Spannkräfte einerseits durch die an den Bedienungsorganen von Hand aufgebrachten Kräfte und das Übersetzungsverhältnis von 1:1 gefühlsmässig kontrollierbar sind und andererseits durch eine Kraftanzeige gleichzeitig eine quantitative Kontrolle durchführbar ist. Diese quantitative Kraftanzeige gewährleistet, dass die medialen und die lateralen Bänder, welche durch die von der linken beziehungsweise rechten Hand aufgebrachten Spannkraft separat gespannt werden, exakt mit derselben Vorspannkraft oder mit gezielt unterschiedlichen Vorspannkräften beaufschlagbar sind.

Die Erfindung wird im folgenden anhand der teilweise schematischen Darstellungen für die Verwendung im menschlichen Kniegelenk noch näher erläutert.

Es zeigen:
Fig. 1 eine Seitenansicht der bevorzugten Ausführungsform der erfindungsgemässen Vorrichtung;
Fig. 2 einen Grundriss der in Fig. 1 dargestellten Ausführungsform der erfindungsgemässen Vorrichtung;
Fig. 3 eine perspektivische Darstellung der in den Fig. 1 und 2 dargestellten Ausführungsform der erfindungsgemässen Vorrichtung eingesetzt im gebeugten Kniegelenk; und
Fig. 4 eine perspektivische Darstellung der in den Fig. 1 und 2 dargestellten Ausführungsform der erfindungsgemässen Vorrichtung eingesetzt im gestreckten Kniegelenk.

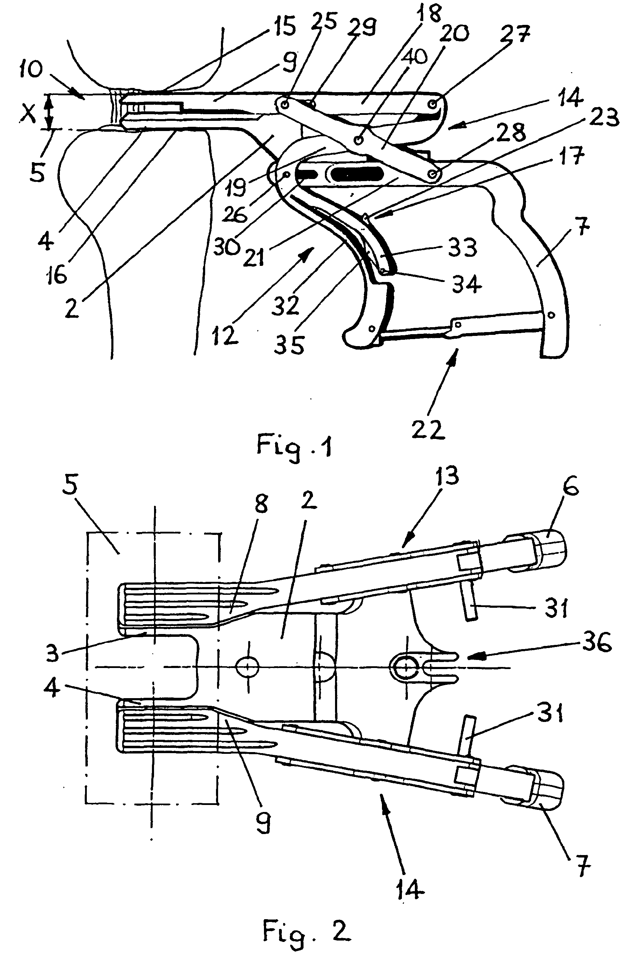

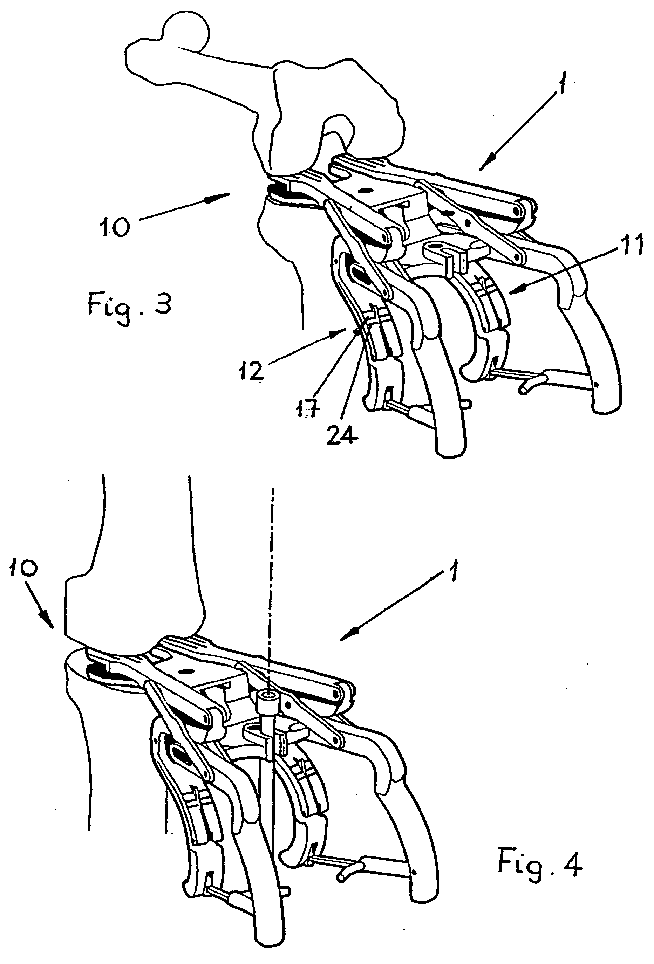

In den Fig. 1 und 2 ist die erfindungsgemässe Vorrichtung 1 mit Spreizmechanismus und dessen Arretierung darstellt. Die Vorrichtung enthält einen plattenförmigen Grundkörper 2, welcher zur sicheren Einleitung der Spreizkraft in die Tibia über eine linke Pratze 4 und eine rechte Pratze 3 mit in einer Ebene 5 liegenden Auflageflächen 16 verfügt. Den Pratzen 3;4 gegenüberliegend sind entsprechend am Grundkörper 2 ein linker Handgriff 7 und ein rechter Handgriff 6 angebracht, welche ein Halten der Vorrichtung mit zwei Händen ermöglichen. Ebenfalls entsprechend zur Anordnung der Pratzen 3;4 und oberhalb der Pratzen 3;4 liegend umfasst die Vorrichtung einen linken Spannhebel 9 und einen rechten Spannhebel 8, welche sich mit ihren Auflageflächen 15 auf dem gegenüberliegenden Anteil des Gelenkes 10 abstützen. Die Spreizwirkung wird durch Betätigen des rechten Handgriffs 6 zusammen mit dem rechten Bedienungshebel 11 für den einen medialen oder lateralen Gelenkanteil erzeugt. Durch Bedienen des linken Handgriffs 7 zusammen mit dem linken Bedienungshebel 12 wird die Spreizfunktion auf den anderen, je nach Gelenk medialen oder lateralen Gelenkanteil erzeugt. Eine linke Paralielverschiebevorrichtung 14 und eine rechte Patallelverschiebevorrichtung 13 gestatten bezüglich der Auflageflächen 16 und den Auflageflächen 15 eine Parallelverschiebung der beiden Spreizerpaare. Die Parallelverschiebevorrichtungen 13;14 sind jeweils als Viergelenk in Form sich kreuzender Stäbe ausgeführt und umfassen je vier Hebel 18;19;20;21, wobei je ein spannhebelseitiger Hebel 18 und ein grundkörperseitiger Hebel 21 parallel angeordnet sind, während je zwei Hebel 19;20 sich kreuzen. Die vier Hebel 18;19;20;21 sind mittels fünf Achsen 25;26;27;28;40 miteinander verbunden. Zwei der Achsen 25;26 sind in den parallelen Hebeln 18;21 in parallel zu den Auflageflächen 15;16 verlaufenden Langlöchern 29;30 verschiebbar gelagert. Diese Ausgestaltung der Parallelverschiebevorrichtungen 13;14 gestattet, dass der spannhebelseitige Hebel 18 und der grundkörperseitige Hebel 21 parallel zueinander oder voneinander weg bewegbar sind. Die Längen der Hebel 18;19;20;21 sind so gewählt, dass bei einer Spannweite X zwischen den Auflageflächen 16 an den Pratzen 3;4 und den Auflageflächen 15 an den Spannhebeln 8;9 zwischen 5 mm und 25 mm das Übersetzungsverhältnis zwischen den manuell an den Handgriffen 6;7 und den Bedienungshebeln 11;12 aufgebrachten Spannkräften und den auf die an das Gelenk 10 (Fig. 3 und 4) angrenzenden Knochen ausgeübten Distraktionskräften 1:1 beträgt.

Zwischen den Handgriffen 6;7 und den Bedienungshebeln 11;12 ist je ein Feststellelement 22 angeordnet, welches in einer beliebigen Stellung der Bedienungshebel 11;12 relativ zu den Handgriffen 6;7 arretierbar ist. Diese Feststellelemente 22 wirken in Form eines Ratschenmechanismus, der sich beim Spreizen zusammenschieben läßt und die erreichte Spreizposition arretiert. Zum Lösen der Arretierposition dient jeweils ein Handhebel 31.

Die Größe der Spreizkraft ist an einer Kraftanzeige 17 mit einer Skala 24 und einem beweglichen Anzeigehebel 23 ablesbar. Bewegt wird dieser Anzeigehebel 23 durch die longitudinale Biegung des einen, durch eine manuell aufgebrachte Spannkraft biegbaren Bedienungshebelteils 32 gegenüber dem anderen gabelartig angeordneten und nicht durch diese Spannkraft beaufschlagten Bedienungshebelteil 33. Der Anzeigehebel 23 ist mittels einer Drehachse 34 im nicht mit der Spannkraft beaufschlagten Bedienungshebelteil 33 gelagert und liegt mit einem Nocken 35 an dem mit der Spannkraft beaufschlagten Bedienungshebelteil 32 auf. Werden mittels der Spannkraft die beiden Bedienungshebelteile 32;33 relativ zueinander bewegt, dreht sich der Anzeigehebel 23 um die Drehachse 34 und auf der Skala 24 wird durch den Anzeigehebel 23 die manuell aufgebrachte Spannkraft angezeigt.

Zur Kontrolle der Achsenverhältnisse am zu versorgenden Gelenk können Richtstäbe (nicht gezeichnet) in einer Führung 36, die winkelfest mit dem Grundkörper 2 verbunden ist, eingelegt werden.

Für die Anwendung im gebeugten Gelenk 10 (Fig. 3) kann durch unterschiedliches Aufspreizen des medialen und lateralen Gelenkanteils eine gezielt unterschiedliche Stabilität der beiden Gelenkanteile erzeugt werden, um eine Rotationsbeweglichkeit, wie sie z.B. für das gebeugte Kniegelenk erwünscht ist, zu erzielen. Diese unterschiedliche Stabilität kann dabei durch definiert unterschiedliche Spreizkräfte medial und lateral erzeugt werden.

Bei Anwendung im gestreckten Gelenk (Fig. 4) kann die Stabilität des Gelenkes in einer exakt definierten Streckposition, z.B. in leichter Hyperextension wie dies z.B. beim Kniegelenk erwünscht ist, exakt eingestellt werden.

## Patentansprüche

1. Vorrichtung zum Spannen von Bändern an nicht-kugeligen Gelenken am menschlichen oder tierischen Körper mit
A) einem prismatischen, zylindrischen oder plattenförmigen Grundkörper (2) mit einer rechten Pratze (3) und einer linken Pratze (4), welche Auflageflächen (16) in einer Ebene (5) aufweisen und damit parallel auf die gelenkseitige Oberfläche eines ersten an ein nicht-kugeliges Gelenk (10) angrenzenden Knochens zur Anlage bringbar sind, sowie einem rechten Handgriff (6) und einem linken Handgriff (7),
B) einem rechten Spannhebel (8) und einem linken Spannhebel (9) mit den Auflageflächen (15), welche parallel zu den Auflageflächen (16) angeordnet sind, wobei zwischen den Auflageflächen (15;16) des rechten Spannhebels (8) und der rechten Pratze (3) eine Spannweite Y und zwischen den Auflageflächen (15;16) des linken Spannhebels (9) und der linken Pratze (4) dieselbe oder eine andere Spannweite X einstellbar ist und die Auflageflächen (15) auf die gelenkseitige Oberfläche eines zweiten an das Gelenk (10) angrenzenden Knochens zur Anlage bringbar sind;
C) einem rechten Bedienungshebel (11) und einem linken Bedienungshebel (12), welche gleichzeitig mit dem Halten der Vorrichtung mit je einer Hand am entsprechenden Handgriff (6;7) einzeln mit der jeweils selben Hand betätigbar sind;
D) einer rechten Parallelverschiebevorrichtung (13) und einer linken Parallelverschiebevorrichtung (14), welche je durch den entsprechenden Bedienungshebel (11;12) antreibbar sind und so mit je einem Spannhebel (6;7) verbunden sind, dass bei einer Bewegung der Bedienungshebel (11;12) die Spannweiten X beziehungsweise Y unabhängig voneinander einstellbar sind,
**dadurch gekennzeichnet, dass**
E) die Parallelverschiebevorrichtungen (13;14) als Viergelenk-Hebelgetriebe ausgebildet sind;
F) jeder Bedienungshebel (11;12) eine Kraftanzeige (17) umfasst; und
G) die Kraftanzeigen (17) so ausgebildet sind, dass beim Spannen der Bänder ein separates Ablesen der auf jeden Bedienungshebel (11;12) ausgeübten Kraft ermöglicht wird.

2. Vorrichtung nach Anspruch 1, **dadurch gekennzeichnet, dass** die Parallelverschiebevorrichtungen (13;14) aus Hebelgetrieben bestehen.

3. Vorrichtung nach Anspruch 2, **dadurch gekennzeichnet, dass** die Hebelgetriebe jeweils vier Hebel (18;19;20;21) umfassen, wobei ein oberer Hebel (18) und ein unterer Hebel (21) parallel angeordnet sind, der obere Hebel (18) mit dem jeweiligen Spannhebel (8;9) verbunden ist, der untere Hebel (21) mit dem Grundkörper (2) verbunden ist und die Verbindungshebel (19;20) das Scherengestänge bilden, so dass der obere Hebel (18) und der untere Hebel (21) parallel zueinander oder voneinander weg bewegbar sind.

4. Vorrichtung nach einem der Ansprüche 1 bis 3, **dadurch gekennzeichnet, dass** die Parallelverschiebevorrichtungen (13;14) bezüglich der Kraftübertragung zwischen der mit den Fingern der jeweiligen Hand auf den entsprechenden Bedienungshebel (11;12) ausgeübten Spannkraft und der von dem entsprechenden Spannhebel (6;7) und der entsprechenden Pratze (3;4) ausgeübten Distraktionskraft auf die an das Gelenk (10) angrenzenden Knochen in einem definierten Arbeitsbereich ein Übersetzungsverhältnis von 1 : 1 gewährleisten.

5. Vorrichtung nach Anspruch 4, **dadurch gekennzeichnet, dass** der mögliche Arbeitsbereich darin besteht, dass die Spannweiten X beziehungsweise Y zwischen den Auflageflächen (16) an den Pratzen (3;4) und den Auflageflächen (15) an den Spannhebeln (8;9) zwischen 5 mm und 35 mm betragen.

6. Vorrichtung nach Anspruch 5, **dadurch gekennzeichnet, dass** der definierte Arbeitsbereich darin besteht, dass die Spannweiten X beziehungsweise Y zwischen den Auflageflächen (16) an den Pratzen (3;4) und den Auflageflächen (15) an den Spannhebeln (8;9) zwischen 7 mm und 12 mm betragen.

7. Vorrichtung nach einem der Ansprüche 1 bis 6, **dadurch gekennzeichnet, dass** die Bedienungshebel (11;12) longitudinal elastisch biegbar sind.

8. Vorrichtung nach einem der Ansprüche 1 bis 7, **dadurch gekennzeichnet, dass** zwischen einem Handgriff (6;7) und dem entsprechenden Bedienungshebel (11;12) teleskopartige und in ihrer Länge lösbar blockierbare Feststellelemente (22) angeordnet sind, mittels welcher die Distanz zwischen der Auflagefläche (16) einer Pratze (3;4) und der Auflagefläche (15) des entsprechenden Spannhebels (8;9) lösbar fixierbar ist.

9. Vorrichtung nach einem der Ansprüche 2 bis 8, **dadurch gekennzeichnet, dass** die Kraftanzeige (17) einen gegenüber einer Skala (24) bewegbaren Anzeigehebel (23) umfasst.

10. Vorrichtung nach Anspruch 9, **dadurch gekennzeichnet, dass** der Anzeigehebel (23) durch die Ausbiegung des entsprechenden Bedienungshebels (11;12) antreibbar ist.

## Claims

1. Device for tensioning ligaments of non-spheroid joints in the human or animal body, with
A) a prismatic, cylindrical or plate-like main body (2) with a right claw (3) and a left claw (4) which have bearing surfaces (16) in a plane (5) and thus can be made to bear in parallel on the joint-side surface of a first bone adjacent to a non-spheroid joint (10), and a right hand grip (6) and a left hand grip (7);
B) a right tensioning lever (8) and a left tensioning lever (9) with the bearing surfaces (15) which are arranged parallel to the bearing surfaces (16), it being possible for a tensioning width Y to be set between the bearing surfaces (15; 16) of the right tensioning lever (8) and the right claw (3), and for an identical or different tensioning width X to be set between the bearing surfaces (15; 16) of the left tensioning lever (9) and the left claw (4), and the bearing surfaces (15) can be made to bear on the joint-side surface of a second bone adjacent to the joint (10);
C) a right operating lever (11) and a left operating lever (12) which, at the same time as the device is being held with one hand on each hand grip (6; 7), can be operated individually using the same hand;
D) a right parallel displacement device (13) and a left parallel displacement device (14) which can each be driven by the corresponding operating lever (11; 12) and are connected to a respective tensioning lever (8; 9) in such a way that, upon movement of the operating levers (11; 12), the tensioning widths X and Y, respectively, can be set independently of each other,
**characterized in that**
E) the parallel displacement devices (13; 14) are four-link lever mechanisms;
F) each operating lever (11; 12) comprises a force indicator (17); and
G) the force indicators (17) being configured such that, upon tensioning of the ligaments, they allow the force exerted on each operating lever (11; 12) to be read off separately.

2. Device according to Claim 1, **characterized in that** the parallel displacement devices (13; 14) consist of lever mechanisms.

3. Device according to Claim 1 or 2, **characterized in that** the lever mechanisms each comprise four levers (18; 19; 20; 21), where an upper lever (18) and a lower lever (21) are arranged in parallel, the upper lever (18) is connected to the respective tensioning lever (8; 9), the lower lever (21) is connected to the main body (2), and the connecting levers (19; 20) form the scissors arrangement so that the upper lever (18) and the lower lever (21) can be moved towards each other or away from each other in parallel.

4. Device according to one of Claims 1 to 3, **characterized in that**, within a defined working range, the parallel displacement devices (13; 14) guarantee a transmission ratio of 1:1 as regards the force transmission between the tensioning force exerted by the fingers of the respective hand on the corresponding operating lever (11; 12), and the distraction force exerted by the corresponding tensioning lever (8; 9) and the corresponding claw (3; 4) on the bones adjacent to the joint (10).

5. Device according to Claim 4, **characterized in that** the possible working range is defined by the fact that the tensioning widths X and Y between the bearing surfaces (16) of the claws (3; 4) and the bearing surfaces (15) of the tensioning levers (8; 9) are between 5 mm and 35 mm.

6. Device according to Claim 5, **characterized in that** the defined working range is set by the fact that the tensioning widths X and Y between the bearing surfaces (16) of the claws (3; 4) and the bearing surfaces (15) of the tensioning levers (8; 9) are between 7 mm and 12 mm.

7. Device according to one of Claims 1 to 6, **characterized in that** the operating levers (11; 12) are longitudinally elastically flexible.

8. Device according to one of Claims 1 to 7, **characterized in that** telescope-like fixing elements (22), which can be releasably locked in position in terms of their length, are arranged between a hand grip (6; 7) and the corresponding operating lever (11; 12), by means of which elements the distance between the bearing surface (16) of a claw (3; 4) and the bearing surface (15) of the corresponding tensioning lever (8; 9) can be fixed in a releasable manner.

9. Device according to one of Claims 2 to 8, **characterized in that** the force indicator (17) includes an indicator lever (23) which can be moved relative to a scale (24).

10. Device according to Claim 9, **characterized in that** the indicator lever (23) can be moved by the bending of the corresponding operating lever (11; 12).

## Revendications

1. Dispositif permettant de tendre des ligaments au niveau d'articulations non-orbiculaires du corps humain ou animal comportant
A) un corps de base (2) prismatique, cylindrique ou en forme de plaque pourvu d'une griffe droite (3) et d'une griffe gauche (4), lesquelles présentent des surfaces d'appui (16) s'inscrivant dans un plan (5) et que l'on peut par conséquent faire reposer de manière parallèle sur la surface - située côté articulation - d'un premier os contigu à une articulation (10) non-orbiculaire, ainsi qu'une poignée droite (6) et une poignée gauche (7),
B) un levier tendeur droit (8) et un levier tendeur gauche (9) présentant des surfaces d'appui (15) qui sont disposées parallèlement aux surfaces d'appui (16), étant entendu qu'il est possible de régler entre les surfaces d'appui (15;16) du levier tendeur droit (8) et de la griffe droite (3) un écartement Y et entre les surfaces d'appui (15;16) du levier tendeur gauche (9) et de la griffe gauche (4) le même écartement ou un autre écartement X, et qu'il est possible de faire reposer les surfaces d'appui (15) sur la surface - située côté articulation - d'un deuxième os contigu à l'articulation (10);
C) un levier de manipulation droit (11) et un levier de manipulation gauche (12), lesquels peuvent être actionnés de manière individuelle avec respectivement la même main, chacune des poignées (6;7) de l'instrument étant tenue respectivement avec une main;
D) un dispositif de déplacement parallèle droit (13) et un dispositif de déplacement parallèle gauche (14), lesquels peuvent être actionnés chacun au moyen du levier de manipulation respectif (11;12) et qui sont reliés respectivement à un levier tendeur (6;7) de telle sorte qu'il est possible, en bougeant le levier de manipulation (11,12), de régler l'écartement X et l'écartement Y indépendamment l'un de l'autre,
**caractérisé en ce que**
E) les dispositifs de déplacement parallèle (13;14) sont réalisés sous forme de mécanisme à leviers à quatre articulations;
F) chaque levier de manipulation (11;12) comprend un indicateur de force (17); et
G) les indicateurs de force (17) sont conçus de telle sorte qu'il est possible, lorsque les ligaments sont en train d'être tendus, de lire séparément la force exercée sur chaque levier de manipulation (11; 12).

2. Dispositif selon la revendication 1, **caractérisé en ce que** les dispositifs de déplacement parallèle (13;14) sont constitués par des mécanismes à leviers.

3. Dispositif selon la revendication 2, **caractérisé en ce que** les mécanismes à leviers comprennent respectivement quatre leviers (18;19;20;21), un levier supérieur (18) et un levier inférieur (21) étant disposés parallèlement, le levier supérieur (18) étant relié au levier tendeur respectif (8;9), le levier inférieur (21) étant relié au corps de base (2) et les leviers de liaison (19;20) constituant un système de tiges croisées en ciseaux de telle sorte qu'il est possible de rapprocher ou d'éloigner parallèlement l'un de l'autre le levier supérieur (18) et le levier inférieur (21).

4. Dispositif selon l'une des revendications 1 à 3, **caractérisé en ce que** les dispositifs de déplacement parallèle (13;14) garantissent, en ce qui concerne la transmission de force entre la force de tension exercée par les doigts d'une main donnée sur le levier de manipulation correspondant (11;12) et la force d'écartement exercée par le levier tendeur correspondant (6;7) et par la griffe correspondante (3;4) sur les os contigus à l'articulation (10) dans une zone de travail définie, un rapport de transmission de 1:1.

5. Dispositif selon la revendication 4, **caractérisé en ce que** la zone de travail possible est déterminée par le fait que les écartements X et Y prévus respectivement entre les surfaces d'appui (16) au niveau des griffes (3;4) et les surfaces d'appui (15) au niveau des leviers tendeurs (8;9) sont compris entre 5 mm et 35 mm.

6. Dispositif selon la revendication 5, **caractérisé en ce que** la zone de travail définie est déterminée par le fait que les écartements X et Y prévus respectivement entre les surfaces d'appui (16) au niveau des griffes (3;4) et les surfaces d'appui (15) au niveau des leviers tendeurs (8;9) sont compris entre 7 mm et 12 mm.

7. Dispositif selon l'une des revendications 1 à 6, **caractérisé en ce que** les leviers de manipulation (11) sont élastiquement flexible dans le sens longitudinal.

8. Dispositif selon l'une des revendications 1 à 7, **caractérisé en ce qu'**entre une poignée (6;7) et le levier de manipulation correspondant (11;12) sont disposés des éléments de blocage (22) télescopiques pouvant être bloqués de manière amovible sur leur longueur, lesquels permettent de fixer de manière amovible la distance entre la surface d'appui (16) d'une griffe (3;4) et la surface d'appui (15) du levier tendeur correspondant (8;9).

9. Dispositif selon l'une des revendications 2 à 8, **caractérisé en ce que** l'indicateur de force (17) comprend un levier indicateur (23) pouvant se déplacer par rapport à une échelle graduée (24).

10. Dispositif selon la revendication 9, **caractérisé en ce qu'**il est possible d'actionner le levier indicateur (23) en faisant fléchir le levier de manipulation correspondant (11;12).
